# EUROPÄISCHE PATENTSCHRIFT

(11) **EP 1 374 914 B1**
(45) Veröffentlichungstag und Bekanntmachung des Hinweises auf die Patenterteilung: **30.03.2011**
(21) Anmeldenummer: 03012767.4
(22) Anmeldetag: 05.06.2003
(51) Int. Cl.: A61L 2/07, A61K 6/10

(54) **Verfahren zur Keimreduktion von Abformmaterialien**
Method for germ reduction of impression materials
Procédé de réduction de germes dans matériaux d'empreintes

(30) Priorität: 25.06.2002 DE 10228420
(43) Veröffentlichungstag der Anmeldung: 02.01.2004
(73) Patentinhaber: Heraeus Kulzer GmbH, 63450 Hanau (DE)
(72) Erfinder: Grunwald, Martin, Dr., 50259 Pulheim (DE); Esser, Birgit, 41515 Grevenbroich (DE)
(74) Vertreter: Kühn, Hans-Christian

(56) Entgegenhaltungen:
- EP-A- 0 164 190
- WO-A-01/89588
- US-A- 2 223 629
- US-A- 4 033 774

## Beschreibung

Die vorliegende Erfindung betrifft ein Verfahren zur Keimreduktion von, insbesondere elastomeren, Abformmaterialien.

Unterschiedliche Arten an Abformmassen sind an sich bekannt (siehe R.G. Craig, Restaurative Dental Materials, The C.V. Moosbe-Comp. St. Louis, Toronto, London, 1980, S. 1979 ff). An derartige Materialien werden insgesamt sehr hohe Anforderungen gestellt (vergleiche K. Eichner, Zahnärztliche Werkstoffe und ihre Verarbeitung, Bd. 1, A. Hüthig Verlag, Heidelberg, 4. Auflage, 1981, S. 45 ff):
1. Angenehmer Geruch, Geschmack und ästhetisches Aussehen.
2. Die Massen dürfen keine toxischen oder irritierenden Bestandteile enthalten.
3. Die Massen müssen eine mehrmonatige Lagerstabilität aufweisen.
4. Die Massen müssen wirtschaftlich herstellbar sein und eine präzise Abformung ergeben.
5. Die Massen müssen leicht zu handhaben sein.
6. Die Härtungscharakteristik muss den klinischen Erfordernissen entsprechen.
7. Die ausgehärteten Massen müssen elastisch sein und dürfen sich nicht unter Zugbeanspruchung bleibend verformen.
8. Die ausgehärteten Massen müssen eine ausreichende Druckfestigkeit besitzen und dürfen nicht brechen.
9. Die ausgehärteten Massen müssen bei Raumtemperatur und normaler Luftfeuchtigkeit solange dimensionsstabil sein, dass in angemessener Zeit exakte Gipsabdrücke hergestellt werden können.
10. Die ausgehärteten Massen dürfen keine Gipsschädigung hervorrufen und müssen mit anderen Abformmassen kompatibel sein.

Aus der Gruppe der verschiedenen Materialien sind elastomere Abformmaterialien besonders vorteilhaft, unter anderem auch auf Grund der gegenüber den nicht elastomeren Abformmaterialien vorteilhaften anwendungstechnischen und mechanischen Eigenschaften.

Es sind verschiedene Arten von elastomeren Abformmaterialien bekannt, wie zum Beispiel Elastomere mit Polymerkettenstruktur, die durch Additionsreaktion abbinden (wie zum Beispiel additionsvernetzende Silicon-Abformmaterialien (sogenannte A-Silicone), die durch eine Hydrosilierungsreaktion von Vinylgruppen an einem polydiorganylgruppenhaltiges Polymer (Vinylpolymere) mit einem SiH-Gruppen enthaltenden Polydiorganosiloxan (SiH-Vernetzer) miteinander reagieren und dadurch ein Elastomer formen, beziehungsweise entsprechende Polyethermaterialien (wie sie zum Beispiel in DE-A1-37 41 575 beziehungsweise DE-A1-38 38 587 beschrieben sind)), oder aber durch Kondensationsreaktion Elastomere bildende Abformmassen, wie zum Beispiel kondensationsvernetzende Silicon-Abform-materialien (sogenannte C-Silicone) oder aber Polyether-Abformmaterialien (wie sie zum Beispiel in DE 101 04 079.2-42 und zum Beispiel in der dort gewürdigten EP 0 269 819 B1 beschrieben werden). Andere häufig verwendete elastomere Abformmaterialien sind solche mit Polyetherketten und Verknüpfung über Aziridinogruppen (wie zum Beispiel in DE-B-17 45 810 beschrieben); ebenso sind Polyetherabformmaterialien mit Acrylat- beziehungsweise Methacrylatgruppen, zum Beispiel aus EP 0 173 085, bekannt. Diese Abformmaterialtypen erfüllen im Wesentlichen die zuvor genannten generellen Eigenschaften für Abformmaterialien und weisen auch gegenüber Materialien mit hohem Wassergehalt (wie zum Beispiel sogenannte Hydrokolloide auf Agar-Agar-Basis und Alginat-Abformmaterialien) verbesserte Lagerstabilitäten und bessere Stabilität bei Desinfektion auf sowie deutlich erhöhte mechanische Stabilität.

In der Regel liegen diese elastomeren Abformmaterialien vor der "Abbindung" (das heißt, Formung der elastomeren Struktur) als Pasten vor, die in der Regel aus zwei Komponenten (häufig als Basispaste und Katalysator- oder Härterpaste bezeichnet) bestehen und zu dem Elastomer nach Vermischen abbinden.

Durch ein Abformmaterial wird in den zuvor genannten medizinischen Anwendungsbereichen der Negativabdruck einer Situation der körpereigenen Geometrie angefertigt, zum Beispiel zur Herstellung eines Ersatzteils oder aus diagnostischen Gründen. Dabei soll die Situation möglichst detailgenau wiedergegeben werden. Das heißt, aus diesem Grund empfehlen sich insbesondere elastomere Abformmaterialien, die nicht nur eine hohe Detailgenauigkeit und Dimensionsbeständigkeit auch bei Lagerung der Abformung aufweisen, sondern sich auch ohne wesentliche Änderung der physikalischen Eigenschaften gut desinfizieren lassen, was insofern von besonderer Bedeutung ist, da die abgeformten Körperpartien eine mehr oder minder intensive Keimbesiedelung aufweisen, die zu einer Kontamination mit diesen Keimen bei nachfolgenden Arbeitsschritten, wie Modellherstellung, Erstellung der restaurativen Arbeit etc., führen und letztlich auch die mit diesen Abformungen und den mit Hilfe dieser Abformungen daraus hergestellten Ersatzteilen beschäftigten Personen erheblich gefährden können. Daher existieren viele Verfahren zur Desinfektion von ausgehärteten Abformungen (zum Beispiel mittels H₂O₂, UV-Bestrahlung, Anwendung von Desinfektionsmitteln (zum Beispiel in F.M. Blair, R.W. Wassell, British Dental Journal, Vol. 180, No. 10, 1996, S. 369 ff. beziehungsweise G.L. Adabo, E. Zanarotti, R.G. Fonseca, C.A. Cruz, Journal of Prosthetic Dentistry 81 (5), 1999, S. 621 ff.) oder γ-Strahlensterilisation von Abformungen (zum Beispiel in J. Setz, U. Benzing, Deutsche Zahnärztliche Zeitschrift, 44, 1989, S. 106 f.)). Alle diese Verfahren werden jedoch an den bereits "abgebundenen", das heißt, elastomeren Abformungen durchgeführt.

In den zuvor genannten Anwendungsbereichen tritt jedoch häufig bei der Abdrucknahme das Problem auf, dass das Abformmaterial im pastösen Zustand mit verletztem Haut- oder Schleimhaut- oder Knochengewebe in Verbindung kommt (zum Beispiel durch Blutungen an der Schleimhaut bei der dentalen Abdrucknahme beziehungsweise bei Abformungen beim Setzen von Implantaten oder aber bei Abformungen bei noch nicht verheiltem Hautgewebe bei der Abformung im Rahmen von epithetischen Versorgungen oder bei Hautabformungen).

Dabei besteht naturgemäß die Gefahr, dass diese mit dem Abformmaterial in Berührung kommenden Körperpartien mit Keimen (wie zum Beispiel Bakterien, Bazillen, Pilze, Hefen, Viren) aus dem Abformmaterial beziehungsweise aus dem Primärpackmittel beziehungsweise von dem zur Applikation benötigten Zubehör (wie zum Beispiel Mischkanülen für Abformmaterialien, die in Doppelkammerkartuschen angeboten werden, oder aber Anmischspatel) kontaminiert werden; diese Keimkontamination kann zu schwerwiegenden gesundheitlichen Problemen führen, die bei Personen mit eingeschränkter Funktion des Immunsystems besonders negative Auswirkungen haben können.

Es hat daher nicht an Versuchen gefehlt, Maßnahmen zu treffen, die diese Gefährdungen beseitigen.

So wurde von D.N. Firtell, D.J. Moore, G.B. Pelleu Jr. in Journal of Prosthetic Dentistry, 1972, S. 419 - 422 ein Verfahren beschrieben, um Alginatpulver mit Ethylenoxidbegasung zu sterilisieren. Diese für pulverförmige Materialien geeignete Methode lässt sich jedoch nicht in einfacher Weise auf Abformmaterialpasten übertragen. Außerdem weisen Alginat-Abformmaterialien, wie bereits zuvor erläutert, gegenüber anderen elastomeren Abformmaterialien nur unzureichende materialkundliche und anwendungstechnische Eigenschaften auf. Da zudem die Handhabung des beschriebenen Pulver: Wasser-Gemisches unkomfortabel ist, hat diese beschriebene Problemlösung keine Verbreitung gefunden.

Daher wurde versucht, einen keimarmen oder sogar sterilen Abdruck durch Zusatz keimtötender Mittel zu den Abformmaterialien zu erzeugen, indem das Keimwachstum verhindernde Mittel den Materialien zugegeben wurden (zum Beispiel DE 37 24 243, JP 07112910, WO 99/15132, WO 00/07546). Diese Art der Problemlösung birgt die Gefahr einer Eigenschaftsveränderung des so ausgestatteten Abformmaterials, und vor allen Dingen haben alle entsprechenden Zusätze den Nachteil, dass dadurch auch die entsprechende Körperpartie kontaminiert wird mit allen damit verbundenen Nachteilen, wie zum Beispiel lokale Reizungen bis hin zu möglichen grundsätzlichen Unverträglichkeiten beziehungsweise allergischen Reaktionen durch die wirksamen Bestandteile. Darüber hinaus haben antimikrobiell wirksame Wirkstoffe in der Regel ein eingeschränktes Wirkspektrum auf spezielle Keime. Von Th. Kaus, A. Sethi wird in ZWR, 110. Jahrgang, 2001, S. 22- 26 die Verwendung eines strahlensterilisierten Abformlöffels und einer strahlensterilisierten Mischkanüle für ein Abformmaterial in einem Kartuschensystem beschrieben, nicht aber die Verwendung eines sterilisierten Abformmaterials.

Ein Beispiel für γ-Strahlenbehandlung von im Dentalbereich anwendbaren Kunststoffen ist durch JP 52013234 B4 gegeben, die dort beschriebenen Materialien sind jedoch nicht als Abformmassen verwendbar.

In US-Patent 4,033,774 wird ein thermoplastisches, nicht elastomeres dentales Abformmaterial beschrieben, das vor Gebrauch in einem Autoklaven sterilisiert werden können soll, ohne dass dazu weitere Details angegeben werden. Eine Problemlösung für eine mögliche Keimreduktion bei elastomeren Abformmaterialien wird dadurch nicht beschrieben.

Die WO 01/89588 A1 offenbart die Sterilisation von bestimmten additionsvernetzenden Siliconen in medizinischen Bereich.

Der Erfindung liegt die Aufgabe zugrunde, ein Verfahren zur sicheren Keimreduktion von zweikomponentigen, zu einem elastomeren Material verntzenden Abformmaterialien, im Dentalbereich insbesondere für pastöse, zweikomponentige, elastomere Abformmaterialien, ohne zusätzliche Beimengungen zu den Abformmaterialpasten bereitzustellen.

Die Aufgabe wird erfindungsgemäß durch die Merkmale des Anspruchs 1 gelöst. Vorteilhafte Ausgestaltungen der Erfindung sind in den Unteransprüchen angegeben. Überraschenderweise wurde gefunden, dass eine Dampfsterilisation, vorzugsweise in einem Autoklaven, das Problem löst, insbesondere, wenn materialverträgliche, temperaturbeständige Packmittel und Zubehörteile Verwendung finden. Es kann dadurch in einem Schritt eine Keimreduktion des (pastösen), zweikomponentigen, zu einem elastomeren Material vernetzenden, Abformmaterials inklusive des Primärpackmittels und des zur Applikation notwendigen Zubehörs erreicht werden.

Dies ist umso erstaunlicher, als durch Vorversuche nachgewiesen werden konnte, dass das Abformmaterial durch die Bedingungen einer Sterilisation durch trockene Hitze (4 h, 160°C) irreparablen Schaden erleidet, so dass keine Vernetzung zu einem Elastomer mehr erfolgen kann.

Das Prinzip der Dampfsterilisation (zur Keimreduktion und/oder Sterilisation) in einem Autoklaven ist an sich bekannt und findet bereits weite Anwendung. Eine ausführliche Verfahrensbeschreibung und typische Sterilisationsbedingungen sind zum Beispiel in der Monographie K.H. Wallhäußer, Praxis der Sterilisation - Desinfektion - Konservierung - Keimidentifizierung - Betriebshygiene, 3. Auflage, Georg Thieme Verlag, Stuttgart - New York, 1984, S. 169 ff. beschrieben.

Erfindungsgemäß sollte eine Dampfsterilisationsdauer von 15 min und eine Temperatur von 121°C nicht unterschritten werden.

Besonders vorteilhaft sind Dampfsterilisationsbedingungen von 25 min Sterilisierzeit bei 2,2 bar Dampfdruck und einer Temperatur von 136°C.

Der große Vorteil des Dampfsterilisationsverfahrens im Autoklaven liegt in der Tatsache begründet, dass derartige Geräte weit verbreitet sind und die entsprechende Sterilisation auch an einem dem medizinischen Personal zur Verfügung gestellten geeigneten Material selbst vorgenommen werden kann. Weitere Vorteile des Verfahrens der Sterilisation mit Dampf im Autoklaven zur Keimreduzierung liegen auch darin begründet, dass keine giftigen oder potentiell gefährlichen Stoffe dem Material zugefügt oder gehandhabt werden müssen, um das erwünschte Ziel zu erreichen.

Erfindungsgemäß werden zweikomponentige, zu einem elastomeren Material vernetzende Abformmaterialien verwendet, die aus der Gruppe ausgewählt sind, die aus Additions-, Kondensations- oder durch Aziridinogruppen vernetzende Polyether-Abformmaterialien und/oder Abformmaterialien mit Vernetzung über Acrylat- oder Methacrylatgruppen besteht.

Besonders vorteilhaft ist es, wenn das pastöse Abformmaterial in ein Packmittel abgefüllt ist, das ausreichend Material für eine Einzelanwendung enthält. Als Packmittel eignen sich zum Beispiel Dosen, Schlauchbeutel, Tuben, Spritzen und insbesondere Doppelkammerkartuschen, da hieraus direkt mittels eines ebenfalls sterilisierbaren Mischers das Material ausgefördert und gemischt werden kann ohne weiteres manuelles Anmischen wie bei der Verwendung von zum Beispiel Tuben oder Dosen als Packmittel.

Erfindungsgemäß sind die Komponenten des Abformmaterials in der Primärverpackung angeordnet und werden mit Zubehör in einer Sterilisierverpackung der Dampfsterilisation unterworfen. Als Primärverpackung eignet sich z.B. eine dampfsterilisationstabile Doppelkammerkartusche mit dampfsterilisationsstabilen Kolben und Verschlüssen und als Zubehör z.B. Mischdüsen.

Bei der Auswahl der Packmittel- und Zubehörmaterialien ist insbesondere darauf zu achten, dass diese bei den Dampfsterilisationsbedingungen nicht geschädigt werden. Die Primärverpackung und das Zubehör sollten bei Dampfsterilisierbedingungen stabil sein, und das Material der Primärverpackung und des Zubehörs sollten die Materialeigenschaften des Abformmaterials und/oder dessen Komponenten nicht beeinflussen.

Bei der Verwendung von Doppelkammerkartuschen (die an sich für diesen Anwendungsbereich bekannt sind) lassen sich bei Dampfsterilisation von Silicon-Abformmaterialien zum Beispiel vorteilhafterweise Kartuschenkörper aus Polypropylen oder Polyamid 6 einsetzen; als Verschlussstopfen empfehlen sich solche aus Polypropylen mit Polyamid 66-Dichtstopfen oder aber Gummidichtscheiben aus dampfsterilisierbaren Gummiqualitäten; als Kolben empfehlen sich insbesondere Doppellippen- oder O-Ring-Kolben aus Polyamid 6 mit Silicon-O-Ringen; grundsätzlich sind aber auch andere dampfsterilisationsbeständige Materialien einsetzbar, soweit diese mit den entsprechenden Abformmaterialklassen kompatibel sind.

Bevorzugt werden gleichzeitig mit dem Abformmaterial eine Primärverpackung des Abformmaterials und/oder dessen Komponenten und/oder Zubehör zum Mischen oder zur Applikation des Abformmaterials mit Dampf behandelt.

In einer typischen Ausführungsform wird das im Primärpackmittel befindliche zweikomponentige pastöse Abformmaterial zusammen mit dem zur Mischung beziehungsweise Applikation benötigten Zubehör in eine einseitig wasserdampfdurchlässige Sterilisierverpackung (die an sich Stand der Technik für Dampfsterilisationsvorgänge im medizinischen Bereich darstellt) eingeschweißt und dann dem Dampfsterilisationsprozess im Autoklaven unterworfen.

In einer bevorzugten Ausführungsform wird ein solches Präzisionsabformmaterial in einer geeigneten dampfsterilisierbaren Doppelkammerkartusche zusammen mit den geeigneten statischen Mischern in eine einseitig wasserdampfdurchlässige Klarsichtsterilisierverpackung eingeschweißt und dem Dampfsterilisationsprozess im Autoklaven unterworfen, wobei direkt ein keimreduziertes Kit für die Anwendung in einer sterilen Umverpackung zur Verfügung steht, die unmittelbar vor Anwendung geöffnet wird, so dass für die Abdrucknahme ein keimreduziertes Material zur Verfügung steht. Die Dampfsterilisation wird bei einer Temperatur von höchstens 138 °C und einem Druck von höchstens 2,3 bar durchgeführt, und die Dauer des Dampfsterilisationsverfahrens beträgt bevorzugt höchstens 30 Minuten.

Es ist unerheblich, ob die Dampfsterilisation unmittelbar vor der Anwendung oder bereits zu einem früheren Zeitpunkt durchgeführt wird. Ebenso ist es möglich, bereits zuvor eine Dampfsterilisation durchzuführen und ein entsprechend behandeltes Material und Zubehör an den Handel beziehungsweise an den Anwender abzugeben. Besonders bevorzugt sind hierbei die in einer Sterilisierverpackung eingeschweißten dampfsterilisierten Kits, da dort bis zum Öffnen der Verpackung durch den Anwender die Keimreduktion für alle bei der Anwendung möglicherweise mit dem Patienten in Berührung kommende Einzelteile erhalten bleibt.

Erfolgt die Dampfsterilisation im Autoklaven unmittelbar vor der Anwendung, sind naturgemäß Abkühlzeiten einzuhalten, damit an dem zu behandelnden Patienten keine Materialien mit einer Temperatur angewendet werden, die wesentlich über Körpertemperatur liegt, um Schädigungen des Patienten zu vermeiden und die materialkundlichen Eigenschaften des Abformmaterials zu gewährleisten. Vorteilhaft ist eine Anwendung, bei der das Abformmaterial Raumtemperatur (18-25°C) aufweist.

Erfolgt die Behandlung durch Dampfsterilisation im Autoklaven bereits zu einem früheren Zeitpunkt, dann sind die Zwischenlagerungsbedingungen entsprechend den Empfehlungen für die Art des Abformmaterials einzuhalten, um keine materialkundlich relevanten Veränderungen zu erfahren.

Ein solches keimreduziertes Material eignet sich darüber hinaus auch hervorragend für die Übertragung von biologischen oder arzneilich wirksamen Strukturen durch entsprechende Stempeltechniken, da durch die Keimreduzierung des angefertigten Stempelmaterials eine deutlich verringerte Gefahr einer Keimeinschleppung in das zu stempelnde Substrat oder aber auf die gestempelten Oberflächenstrukturen insbesondere bei Handhabung biologischen oder von arzneilich wirksamen Materialien besteht. Diese auch als micro contact printing bekannten Stempeltechniken sind an sich bekannt und werden in den wesentlichen Eigenschaften zum Beispiel in Y. Xia, G.M. Whitesides, Angew. Chem. Int. Ed. 1998, 37, 550 - 575, beschrieben.

Die Erfindung betrifft die Verwendung des vorgestellten Dampfsterilisationsverfahrens für im Dentalbereich verwendete Abformmassen.

Die nachfolgenden Beispiele sollen die Erfindung erläutern, ohne sie jedoch einzuschränken. Wenn nicht anders angegeben, wurde die Keimreduktion durch Einschweißen der Abformmassen im Primärpackmittel inklusive Zubehör in eine einseitig wasserdampfdurchlässige Klarsichtsterilisierverpackung (zum Beispiel MELAfol^{®} der Firma MELAG oHG) und anschließende Dampfsterilisationsbehandlung bei 25 min Sterilisierzeit bei 136°C und 2,2 bar Wasserdampfdruck in einem Autoklaven (zum Beispiel Typ 29 der Firma MELAG oHG) durchgeführt.

### Beispiel 1 (Vergleichsbeispiel):

Eine handelsübliche dentale Abformmasse auf Basis eines additionsvernetzenden Silicons (A-Silicon) (Flexitime Mono Phase, in temperaturstabile Doppelkartuschen abgefüllt) wird einem Sterilisationsprozess durch trockene Hitze (4h, 160°C) unterworfen.
Nach dem Abkühlen auf Raumtemperatur war das Material nicht mehr in der Lage, zu einem elastomeren Formkörper zu vernetzen.

### Beispiel 2 (Vergleichsbeispiel)

Eine pastöse, zweikomponentige handelsübliche A-Siliconabformmasse (Provil Novo Medium C.D. 2) wird in eine Doppelkammerkartusche mit Kolben und Einlegedichtung der Verschlusskappe auch Polyethylen (nicht dampfsterilisierbar) gefüllt und einem Dampfsterilisationsprozess unterworfen. Kolben und Verschluss waren geschmolzen, das Material war dadurch nicht anwendbar. Die materialkundlichen Eigenschaften haben demgegenüber durch den Dampfsterilisationsprozess keine wesentliche Änderung erfahren.

### Beispiel 3 (nicht erfindungsgemäß):

Eine handelsübliche dentale Abformmasse auf Basis eines additionsvernetzenden Silicons (Flexitime Mono Phase in temperaturstabilen Doppelkammerkartuschen) wird zum einen ohne vorhergehende Behandlung und zum anderen nach Keimreduktion durch Dampfsterilisation physikalisch und anwendungstechnisch geprüft.
Ergebnis der physikalischen Prüfung:

| | | Nicht behandelt | Nach Dampfsterilisationsprozess |
|---|---|---|---|
| Viskosität Basis | [Pas] | 197 | 411 |
| Viskosität Katalysator | [Pas] | 164 | 298 |
| Verarbeitungszeit | [min] | 3,55 | 4,30 |
| Rückstellung nach Verformung | [%] | 99,4 | 98,4 |
| Shore A-Härte nach 10 min | | 58 | 60 |
| Dimensionsänderung | [%] | 0,19 | 0,16 |

Die anwendungstechnische Prüfung zeigt keine wesentliche Eigenschaftsänderung durch den Dampfsterilisationsvorgang.

Die Abformmasse entspricht auch nach dem Dampfsterilisationsprozess noch voll den für dentale Abformungen notwendigen Eigenschaften und den Anforderungen der EN 24823.

### Beispiel 4 (nicht erfindungsgemäß):

Es wurde ein additionsvemetzendes Siliconabformmaterial (Flexitime Mono Phase) und ein Zubehörteil (Mischdüse) vor und nach dem Dampfsterilisationsprozess einer mikrobiologischen Prüfung unterworfen (Steriltest).

| | ohne Dampfsterilisationsprozess | | nach Dampfsterilisationsprozess | |
|---|---|---|---|---|
| | Keimzahl Bakterien [KBE/Prüfmuster] | Keimzahl Pilze, Hefen [KBE/Prüfmuster] | Keimzahl Bakterien [KBE/Prüfmuster] | Keimzahl Pilze, Hefen [KBE/Prüfmuster] |
| Mischdüse | 30 | 10 | Keine vermehrungsfähigen Mikroorganismen nachweisbar | Keine vermehrungsfähigen Mikroorganismen nachweisbar |
| | | | | |
| A-Silicon-Abformmasse (Flexitime Mono Phase)* | < 10 | <10 | Keine vermehrungsfähigen Mikroorganismen nachweisbar | Keine vermehrungsfähigen Mikroorganismen nachweisbar |

| | | | | |
|---|---|---|---|---|
| * In Prüfansätzen (Validierung der Prüfung auf Sterilität (Direktbeschickung) (gemäß EP Suppl. 2001)) erfolgte das Wachstum von Mikroorganismen (Bacillus subtilis, Pseudomonas aeruginosa, Clostridium sporogenes, Staphylococus aureus, Candida albicans, Aspergillus niger) genauso üppig wie in Referenzansätzen (die Validierung der Methode ist damit gültig). | | | | |

### Beispiel 5 (nicht erfindungsgemäß):

Eine pastöse, zweikomponentige A-Siliconabformmasse wird in eine Polypropylen-Doppelkammerkartusche mit nicht dampfsterilisationsbeständigen Verschlüssen und Kolben und solchen mit dampfsterilisierbaren Kolben eingefüllt, desgleichen in eine Polyamid-Doppelkammerkartusche mit dampfsterilisierbaren Kolben und Verschlüssen. Die unbehandelten und nach dem Dampfsterilisationsprozess geprüften Materialien wurden nach einem Monat nochmals vermessen.
In analoger Weise wurde ein durch Kondensationsreaktion vernetzendes zweikomponentiges pastöses Polyether-Abformmaterial vor und nach Dampfsterilisationsprozess getestet.
Die Daten der Prüfungen sind der nachfolgenden Tabelle zu entnehmen.

| **Material** | **A-Silicon-Abformmaterial, Flexitime Mono Phase 1:1** (**nicht erfindungsgemäß)** | | | | **Polyether-Abformmaterial** mit **kondensationsvernetzenden Eigenschaften (4:1)** | |
|---|---|---|---|---|---|---|
| Packmittel | PP-Kartusche mit nicht temperaturbeständigem Doppellippenkolben und nicht temperaturbeständiger Verschlusskappe | temperaturfestes Doppelkartuschen-System (PA) mit temperaturstabilen Kolben und Verschlüssen | | PP-Kartusche mit temperaturbeständigem Kolben und temperaturbeständiger Kappe | temperaturfestes (1:1) Kartuschen-System (Komponenten wie bei Flexitime Mono Phase) | Standard (4:1)-CD-System (nicht temperaturbeständige Kolben und Verschlusskappe) |
| angemischt mit | grüner Mischdüse, unsteril | grüner Mischdüse, unsteril | dampfsterilisiertem grüner Mischdüse | dampfsterilisiertem grüner Mischdüse | Handanmischung | (4:1) Mischdüse |
| Behandlung | ohne, "O-Probe"/ (nach 1 Monat 30°C) | Unbehandelt/ (nach 1 Monat 30°C) | dampfsterilisiert (25 min/136°C/2,2 bar)/ (nach 1 Monat 30°C) | dampfsterilisiert (25 min/136°C/2,2 bar)/ (nach 1 Monat 30°C) | dampfsterilisiert (25 min/136°C/2,2 bar) | unbehandelt ("O-Probe") |
| Mundverweildauer [min] | 2,5 | 2,5 | 2,5 | 2,5 | 3,0 | 3,5 |
| Viskosität Basis [Pas] | 193/(210) | 223/(217) | 542/(387) | 526/(388) | 193 | 85 |
| Viskosität Katalysator [Pas] | 170/(200) | 174/(197) | 387/(308) | 350/(256) | 48 | 90 |
| Verarbeitungszeit (OSC-Messung)[min] | 2,50/(2,55) | 2,55/(2,75) | 2,701(2,80) | 2,601(2,60) | 1,80 | 1,65 |
| Abbindezeit (OSC-Messung)[min] | 3,20/(3,40) | 3,40/(3,65) | 3,60/(3,85) | 3,451(3,55) | 3,55 | 2,75 |
| Rückstellung nach Verformung | 98,95/(nicht bestimmt) | nicht bestimmt | 98,551(nicht bestimmt) | 98,581(nicht bestimmt) | - | - |
| Shore A, 10 min | 57/(nicht bestimmt) | nicht bestimmt | 58/(nicht bestimmt) | 59/(nicht bestimmt) | - | - |
| Shore A, 1 h | 58/(nicht bestimmt) | nicht bestimmt | 59/(nicht bestimmt) | 59/(nicht bestimmt) | - | - |
| Dimensionsänderung [%] | 0,25/(nicht bestimmt) | nicht bestimmt | 0.27/(nicht bestimmt) | 0.30/(nicht bestimmt) | - | - |

Es lässt sich festhalten, dass bei den A-Silicon-Abformmassen durch den Dampfsterilisationsprozess die Rückstellung nach Verformung, die Shore A-Härte und die Dimensionsänderung sich nicht wesentlich geändert haben. Wie in Beispiel 2 ebenfalls zu erkennen war, erhöhen sich durch die thermische Behandlung die Viskositäten (was durch das enthaltene Strukturmittel bedingt ist); dieser Vorgang ist aber zumindest teilweise reversibel, wie die 1-Monats-Werte zeigen. Außer einer geringfügigen Verlangsamung ist kein negativer Effekt auf die Vernetzungskinetik festzustellen. Das Material ist in den Packmitteln mit und ohne zuvor erfolgte Dampfsterilisation lagerstabil, wie die Nachmessungen nach einem Monat zeigen.
Das Polyetherabformmaterial zeigt ebenfalls keine wesentliche Beeinträchtigung der physikalischen Parameter. Auch nach dem Dampfsterilisationsprozess waren die A-Silicon-Abformmasse und die Polyether-Abformmasse anwendungstechnisch akzeptable Abform-materialien.

### Beispiel 6 (Erfindung):

An einer durch Kondensationsreaktion vernetzenden Polyether-Abformmasse wurde nach dem durchgeführten Dampfsterilisationsprozess Keimzahlen bestimmt mit dem Ergebnis, dass keine vermehrungsfähigen Mikroorganismen nachweisbar waren.

### Beispiel 7 (Erfindung):

Handelsübliches, zweikomponentiges Polyethermaterial (Impregum Garant L Duo Soft in Doppelkammerkartuschen, 2:1), vernetzend über Aziridinogruppen, wurde in einer dampfsterilisationsbeständigen Doppelkartusche einem Dampfsterilisationsprozess unterworfen und danach mit dem unbehandelten Material vergleichend physikalisch geprüft.

| | Aziridinopolyetherabformmasse (unbehandelt) | Aziridinopolyetherabformmasse (nach Dampfsterilisationsprozess) |
|---|---|---|
| Viskosität Basis [Pas] | 83 | 121 |
| Viskosität Katalysator [Pas] | 148 | 176 |
| Verarbeitungszeit (OSC-Messung) (nach Handanmischung 30 s) [min] | 2,50 | 2,00 |
| Abbindezeit (OSC-Messung) (nach Handanmischung 30 s) [min] | 3,50 | 2,80 |
| Rückstellung nach Verformung (nach Handanmischung 30 s) [%] | 98,6 | 98,6 |

Trotz der beobachteten Veränderungen bei Viskosität und Vernetzungskinetik genügen die Materialeigenschaften nach dem Dampfsterilisationsprozess noch den materialkundlichen und anwendungstechnischen Anforderungen.

## Patentansprüche

1. Verfahren zur Keimreduktion von zweikomponentigem, zu einem elastomeren Material vernetzendem Abformmaterial im Dentalbereich, das aus der Gruppe ausgewählt ist, die aus (a1) additionsvernetzenden Polyether-Abformmaterialien, (a2) kondensationsvernetzenden Polyether-Abformmaterialien, (a3) durch Aziridinogruppen vernetzenden Polyather-Abformmaterialien, (a4) Abformmaterialien, die über Acrylatgruppen vernetzen und (b5) Abformmaterialien, die über Methacrylatgruppen vernetzen, besteht, bei dem man
(a) eine Sterilisierverpackung bereitstellt, die eine Primärverpackung und Zubehör enthält, wobei in der Primärverpackung die Komponenten des zu einem elastomeren Material vernetzenden Abformmaterials angeordnet sind, und
(b) eine Dampfsterilisation bei einer Temperatur von wenigstens 121°C und höchstens 138°C für eine Dauer von wenigstens 15 Minuten und höchstens 30 Minuten und einem Druck von höchstens 2,3 bar durchführt.

2. Verfahren nach Anspruch 1, **dadurch gekennzeichnet, dass** die Primärverpackung eine dampfsterilisationsstabile Doppelkammerkartusche mit dampfsterilisationsatabilem Kolben und Verschlüssen ist und das Zubehör Mischdüsen sind.

3. Verfahren nach Anspruch 1 oder 2, **dadurch gekennzeichnet, dass** das Abformmaterial zur Herstellung von Stempeln zur Übertragung von Strukturen, insbesondere von biologisch und/oder arzneilich wirksamen Substraten, verwandt wird.

## Claims

1. Method for germ reduction for two-component impression material for dental applications that cross-links into an elastomeric material and is selected from the group consisting of (a1) polyether impression materials cross-linking by means of addition, (a2) polyether impression materials cross-linking by means of condensation, (a3) polyether impression materials cross-linking by means of aziridino groups, (a4) impression materials cross-linking by means of acrylate groups, and (b5) impression materials cross-linking by means of methacrylate groups, in which
(a) a sterilising packaging is provided that contains a primary packaging and accessories, whereby the components of the impression material that cross-links into an elastomeric material are arranged in the primary packaging, and
(b) a steam sterilisation is carried out at a temperature of at least 121 °C and at most 138°C for a duration of at least 15 minutes and at most 30 minutes and at a pressure of at most 2.3 bar.

2. Method according to claim 1, **characterised in that** the primary packaging is a steam sterilisation-resistant double-chamber cartridge with steam sterilisation-resistant plunger and closures and the accessories are mixing nozzles.

3. Method according to claim 1 or 2, **characterised in that** the impression material is used for manufacturing punches for the transfer of structures, in particular of substrates with biological and/or pharmaceutical activity.

## Revendications

1. Procédé de réduction des germes de matériau de moulage bicomposant se réticulant en un matériau élastomère dans le domaine dentaire, qui est sélectionné parmi le groupe qui se compose de (a1) matériaux de moulage de polyéther à réticulation par addition, (a2) matériaux de moulage de polyéther à réticulation par condensation, (a3) matériaux de moulage de polyéther à réticulation par des groupes aziridino, (a4) matériaux de moulage qui se réticulent par le biais de groupes acrylate et (b5) matériaux de moulage qui se réticulent par le biais de groupes méthacrylate, dans lequel on
(a) met à disposition un emballage de stérilisation qui contient un emballage primaire et des accessoires, dans lequel les composants du matériau de moulage se réticulant en un matériau élastomère sont disposés dans l'emballage primaire, et
(b) réalise une stérilisation à la vapeur à une température d'au moins 121°C et de maximum 138°C pendant une durée d'au moins 15 minutes et de maximum 30 minutes et une pression de 2,3 bars maximum.

2. Procédé selon la revendication 1, **caractérisé en ce que** l'emballage primaire est une cartouche à double chambre stable à la stérilisation à la vapeur avec des pistons et fermetures stables à la stérilisation à la vapeur et les accessoires sont des buses mixtes.

3. Procédé selon la revendication 1 ou 2, **caractérisé en ce que** le matériau de moulage est utilisé en vue de fabriquer des empreintes pour le transfert de structures, notamment de substrats actifs du point de vue biologique et/ou médicamenteux.
